# EUROPEAN PATENT APPLICATION

(11) **EP 2 211 296 A1**
(43) Date of publication of application: **28.07.2010**
(21) Application number: 09000743.6
(22) Date of filing: 21.01.2009
(51) Int. Cl.: G06Q 10/00, G06F 19/00

(54) **Specially triggered electronic communication messages**

(71) Applicant: The Last One GmbH, 34246 Vellmar (DE)
(72) Inventor: Bolsenkötter, Frank, 34246 Vellmar (DE)
(74) Representative: Reinhardt, Thomas Johannes

(57) **Abstract**

The present invention relates to the field of electronic message communications via an electronic network wherein the sending of messages is triggered by some event signal. In order to provide a more flexible communication method enabling more and different diverse uses, it is particularly proposed to pre-store individual, personal messages of an author person at a portal server, and send them automatically without intervention of the author person being required, to respective pre-stored recipient persons after a signal has come in which is related to said author person. The signal may encode different pre-defined life situations:
1) a signal generated by elapse of a pre-programmed timer of a network device located remote from a respective sender server computer and associated solely with a single author, wherein said network device is by-itself able to establish a communication channel to said server and to transmit said event signal to said server along with a pre-programmed ID of said author person,
2) a signal generated by an authority, in particular by the police in case of an accident in which said author person is involved,
3) a signal generated by a mortician network or by a physician telling that said author person has been found dead,
4) a signal generated by a bank telling that a predefined amount of money has been drawn from or credited to a bank account of said author person.

## Description

### 1. BACKGROUND OF THE INVENTION

### 1.1. FIELD OF THE INVENTION

The present invention relates to the field of electronic message communications via an electronic network,
and it specifically relates to a method and system according to a respective preamble of the independent claims.

### 1.2. DESCRIPTION AND DISADVANTAGES OF PRIOR ART

Electronic messages are distributed in prior art via an electronic network in a broad diversity of different types and implementations. For example, the registered clients of an Internet Service Provider are informed about the new features of a certain product by e-mail messages. They are sent out automatically by a batch process triggered for example by a timer of an e-mail server computer which starts the sending process for example at a predetermined clock, for example at 3.00 a.m. in a period of assumable low network load. Here, the trigger signal is given by a timer which has a central function and location in relation to a plurality of different remote recipients of the email messages.

A disadvantage of this method is that the definition of the trigger event which triggers the sending of the messages is not flexibly usable for different use cases other than existing in prior art. Further, the timer device must be network-connected to the same server computer, i.e. the server which is responsible for sending the messages. This boundary condition restricts the usability of prior art.

A further disadvantage in general prior art of automatically sending messages is that there are used only very limited types of trigger signals, which makes respective communication methods have a respective strictly limited field of use, only. So, those methods are useful in prior art, as the sending work is done automatically, and without major human interference as long as the content of the messages is prepared and the respective addresses of the recipients are pre-defined. Disadvantageously, however, those prior art methods are not enough flexibly applicable and their usage, i.e. the setting of content and recipient, and date and time of sending of the messages is quite a clumsy work, and basically undoable for people who are less computer-minded. As a consequence, prior art automatic sending of messages is not applicable for a broader field of use cases.

### 1.3. OBJECTIVES OF THE INVENTION

It is thus an objective of the present invention to provide a more flexible communication method enabling more and different diverse uses.

### 2. SUMMARY AND ADVANTAGES OF THE INVENTION

This objective of the invention is achieved by the features stated in enclosed independent claims. Further advantageous arrangements and embodiments of the invention are set forth in the respective subclaims. Reference should now be made to the appended claims.

According to its basic aspect the present invention discloses a communication method implemented for example in a portal server computer coupled to an electronic network, for sending a message of a respective message author person to at least one message recipient in response to an incoming event signal associated with said author person, wherein the event signal is for example an electronic mail, or SMS or just a sequence of bits predefined to have the meaning of an event signal associated with said author person, comprising the steps of:
a) prior to the income of said event signal, storing said message of said author person along with a respective address of at least one addressee acting as a future recipient (person) of said message, wherein the message is advantageously composed by said author person,
b) waiting for receiving said event signal, wherein said event signal triggers the start of sending said pre-stored message,
c) receiving said event signal accompanied with an ID of said author person,
d) evaluating said event signal and said ID for retrieving said message from a respective storage device, and
e) sending said message to said recipient,
f) said method being **characterized by** said event signal being selected to be one of the group of:
   f1) a signal generated by elapse of a pre-programmed timer of a network device located remote from a respective sender server computer and associated solely with a single author, wherein said network device is by itself able to establish a communication channel to said server and to transmit said event signal to said server along with a pre-programmed ID of said author person,
   f2) a signal generated by an authority, in particular by the police in case of an accident in which said author person is involved,
   f3) a signal generated by a mortician network or by a physician telling that said author person has been found dead,
   f4) a signal generated by a bank telling that a predefined amount of money has been drawn from or credited to a bank account of said author person.

So, in other words, it is particularly proposed to pre-store individual, personal messages of an author person at a portal server, and send them automatically without intervention of the author person being required, to respective pre-stored recipient persons after a signal has come in which is related to said author person. The signal may encode different pre-defined life situations as indicated above with f1) to f4).

This method can further be enriched by the steps of:
a) storing a parcel of, i.e., composed by said author person, along with a respective address of an addressee acting as a future recipient of said parcel,
b) waiting for receiving said predefined event signal associated with said author person, wherein said event signal triggers the start of sending said parcel,
c) receiving said event signal accompanied with the ID of said author person,
d) evaluating said event signal and the ID, and
e) sending said parcel to said recipient.

According to a basic aspect of the present invention the signals, which trigger the sending of the message or the sending of a plurality of predefined messages, are generated remote from the message sending server and the signals are generated due to the occurrence of an event which in turn is basically not influenced by the author of the respective message to be sent over the network.

So, for example a signal is generated by elapse of a pre-programmed timer of a network device which is located remote from the respective sender server computer and which is associated solely with a single author person, wherein the network device is able to establish automatically a communication channel to the sender server and automatically transmit an event signal to the sender server along with a predefined ID of a respective author person.

Such timer can for example be built-in in a mobile phone acting as the before mentioned network device. When then for example a message is predefined having a highly sensitive content, for example indicating the address of a murderer searched by the police and the author being a potential witness of the respective murder, then the inventional automatic sending of the sensitive content within an email to for example the police can be triggered just by elapse of a timer built-in within the mobile phone in case the witness of the murder is threatened by the murderer. Thus, even if the murderer kills the witness, the murderer cannot prevent the mobile phone from sending the sensitive content to the police when - and this is assumed to be the case - the mobile phone is hidden in a save place and programmed according to prior art to send the sensitive message to the police as indicated by a pre-programmed message address.

The message itself can be for example a short message service or the device can be a notebook implementing a batch program according to prior art which sends away the message if the sending is not prevented manually from a person which is authorized to do that. This can also help any private person from being high-jacked in case the high-jacked person would loose its "value" for the highjackers, if such message would be sent for example to the police.

Or, in case one predefined type of message is predefined to describe that the author person is involved in an accident, and the personal ID of the author is for example his passport number, then the police just needs to enter this passport number into a respective predefined communication form, send this form to the server computer and the server computer would then send out one predefined message to the wife or husband or the relatives of the author informing him that something had gone wrong with him. Then it is easy to just answer this message in order to request for further details. Then the police may in turn send an accident report enriched by further details about the person and the accident. Advantageously the workflow is nearly fully automatically running and so the police is able to process many "accident notifications" in a relatively short time.

Or, according to a further basic aspect of the present invention, let us assume that the author person has died unexpectedly due to an accident or whatever reason. Then of course a physician will determine that the author person is definitely dead and will inform a mortician. The mortician can then use his personal network and may generate a trigger signal in the sense of the present invention including the ID of the dead author person and telling that the respective author person has been found dead. In this case a couple of very useful things can be performed, if the respective author person which had recently died has prepared in advance certain messages, which are only to be sent out by the sender server computer in case such mortician signal comes in.

A "personal network of a mortician" basically exists for every professional mortician, at least in the majority of countries. The mortician knows to interpret the passport data and has for each country a telephone contact, or an e-mail contact address, where to notify the death of a person having occurred. Also national embassies are used as contact addresses in those cases.

The mortician network includes secure communication hardware and software of an Internet-coupled Personal Computer, telephone/ telefax capabilities in order to receive a copy of the death certificate. The mortician gets the full name and residence of the dead person from a copy of the death certificate delivered by a physician who actually determined the physical death of a person. The Identification Card number or Passport number may also be included in the death certificate. If not, the mortician retrieves the Identification Card number via a secure communication channel of its network from the authority which is responsible for management of such data.

The inventional method when used in context with the signal generated by the mortician network includes preferably a user registration procedure which can be handled online via the internet. In addition, the user must preferably identify himself to the portal by performing an officially acknowledged personal identification procedure such as for example in Germany the so-called "Post-Ident" procedure. Alternatively, such identification can also be performed by a trusted person visiting the user in case, the user is for example not able to go to the post and perform the before mentioned "Post-Ident" procedure, for example due to a disease or disability to walk etc.

The before mentioned "Post-Ident" procedure is disclosed in
http://www.deutschepost.de/dpag?xmlfile=1015469

After a user has successfully performed his/her registration, he/she may deposit predefined electronic messages addressed to predefined people. These electronic messages can have text or image or audio or video content. Further, according to a separate advantageous aspect of this embodiment of the inventional method a user may also deposit physical things, preferably readily packed in a parcel or letter envelope including the address of the desired recipient of the physical things contained therein. The messages may be for example personal letters including personal contents which has a particular meaning in relation to the author person and the recipient person. Further, a personal present can be contained in the parcel etc.

The inventional method according to this lastly mentioned feature including a signal generated by a mortician network or a physician telling that the author person has been found dead has a plurality of significant advantages as compared to prior art, wherein in this case a special kind of closest prior art has to be regarded as follows:

Due to the fact, that a personal message is generally sent by the author person by personally triggering the send-procedure, the closest prior art in this special case is that the author person has prepared some letter to a desired recipient and has put such letter at any save place. Then, in case the author person is dead he cannot send away such letter. Instead the author person must rely to a closely related relative such as wife or husband or a good friend or a notary public that those persons will find this letter and send this letter to the desired recipient. The particular disadvantage of this closest prior art is that the author person cannot be sure that the letter will be sent away because there is a risk that such close relative person is curious and opens the letter for reading its content before sending it away. If the sending of the content is related with disadvantages to the curious person, it may be will not send it away. In case the letter is deposited at a notary public, the risk is that this notary will not be or will quite late be informed about the fact that the author person has died. So the author risks that the letter deposited at the notary public will be sent away not at all or at least quite retarded in time.

In relation to these particular disadvantages of closest prior art the inventional method according to this special aspect overcomes these disadvantages because once the mortician network has generated the signal and the signal has been communicated to the portal server the sending of the message or that of the predefined letter will be done automatically without any human intervention by a person who has a personal relation to the dead author person.
Spoken in technical terms, no need of human interventions means that no accompanying actions are necessary when executing the notification procedure. Accompanying actions are in prior art: writing a letter, transposing the thoughts of the dead person in own words, searching the address of the desired recipients and forwarding the dead person's individual, person-specific last will messages to these individual recipient persons.

So, electronic messages and optionally physical things including also hand-written letters or letters at least signed manually with "blue ink" are delivered automatically, without any such accompanying actions and without any regard to personal subjective doubts of nearest relatives. Those relatives simply play no role as they are definitely not needed for sending away the messages.

In this respect, the author person may manage the distribution of stocks of his bank depot, of cash money available at his bank account under those persons who are entitled by law to receive some portion thereof, and to other persons at his free own will present before the author person died, as long as the personal names, addresses and bank account data of these persons are pre-stored at the portal server. Optionally, a lawyer - again his address pre-stored as a trusted person of the author person - is automatically informed and introduced into the distribution procedure in order to control its legality, before the stocks or cash money are actually transferred. Also physical things of major economic or idealistic value and forming part of the inheritance of the author person can be offered to any person as desired by the author. In the respect of distributing stocks and cash money a particular electronic program interface can be implemented between the notification server or the delivery servers and the respective bank in order to implement an automatic processing of the distribution via online transactions. Also an electronic copy of the death certificate is added into the workflow prior to each bank transaction process where this is required by law.
In order to do that the portal server optionally receives from the mortician network an electronic copy of the death certificate associated with a dead author person along with the initiating mortal event signal and the author ID.

So, as a person skilled in the art may appreciate the sending of the message is basically not able to be prevented by a mistrustful person who is going to be fraudulent by reading-the content the letter which is not intended to this person and who would prevent such content to get known by the originally desired person.

A further advantage of this embodiment is that any author person has permanently facilities at disposition to change the content of such messages to be sent away after he has died.

A further advantage of this embodiment is that the messages are sent away quite shortly after the death of the author person has been detected because generally the mortician networks must communicate any facts directly related to a mortician event, i.e. the death event of a person to the family members of the death or to the local authority of the seat of the dead person.

According to a further advantage of this specific embodiment these "death-related" messages can be deposited at a save place - save from foreign access of suspicious persons - from nearly any location in the world as long as there is access to the internet or a comparable network from this location. Also in this context, such access is usable for generating new messages or for updating or deleting existing messages. This is of particular significance in those cases, in which the author person is seriously diseased and is concurrently not at home, where according to the closest prior art would be the prior art "safe place" where to deposit prior art death-related letters.

Further advantageously the access to_generate delete or update messages is possible during basically 365 days a year and 24 hours a day, in contrast to prior art deposition of messages at a notary public office.

Further advantageously the death related messages are safe from to be destroyed by above mentioned suspicious fraudulent persons such as inheritors, adversaries, or materially damaged by fire or accidents. Material destruction of messages is at least excluded according to this embodiment for electronic messages due to repeatedly performed back up procedures and nearly excludable also for physical messages in form of letters or parcels or the like due to the fact, that this physical messages are deposited very carefully in a fire-safe and burglary-save place.

Further advantageously this inventional notification method may act extremely fast compared to prior art. In prior art, the physical deposition must first be known to the inheritor person and in a second step must be accessible by the inheritor person and thirdly must be understood by the inheritor person in order to make him know that he is now obliged to send away the death related letters. These are extensive requirements.

Further advantageously the inventional method provides a written confirmation on each deposited message to the author person.

According to a separate advantageous aspect of the inventional method the author person may also generate physical messages in cooperation with a notary public and may then deposit these particular messages with the knowledge of the notary public at the deposition place of the portal. Then, assumed the author person has died, the notary public can monitor the desired sending of these particular death-related messages.

Further advantageously, the privacy of the content of the death related messages can be guaranteed to the author person. For example, if the author person is criminal he may confess his crimes to the police by means of the inventional method, independently of eventual intervention of his family who would probably, when compared to prior art, certainly first open a letter addressed to the local police station and read the content of the letter before sending the letter actually to the police station. Certainly there may exist many cases where the family does not send away such confession letter to the police, because any good reputation of the family would stand at risk if the police knows the content of such confession letter. So, the inventional method may contribute just by implementing technical means to clear up crime which otherwise would be never cleared up.

Further advantageously the death related messages are deposited safely without any risk to be falsified by other persons. In the near environment of the author person.

Further advantageously the inventional method according to this embodiment is quite easy to handle compared to prior art as long as the author person has once registered successfully. The inventional method will work in a very stable and robust way with a minimum of external influences and risks. Finally, at least, when compared with the deposition of death related messages at a notary public, the costs related to the inventional embodiment can be expected to be significantly lower.

Further advantageously according to this last mentioned inventional embodiment relating to death related messages also electronic messages such as messages of the short messages system (SMS), multimedia messages (MMS), facsimile letters, audio or video recordings are not only able to be deposited but also to be automatically transmitted to the desired recipient person or persons. Remark that in prior art a dead person would never be able to send away such message.

Further advantageously the inventional method according to this embodiment including death related messages can be used to unsubscribe automatically the author person from chatrooms or internet forums etc.

Further advantageously this embodiment can be used in order to delay the publication of messages, even of messages having a larger extent such as a book manuscript, diary manuscript or memoir manuscript.

Further, depending on the legal situation in a respective country the author person can issue any declaration of intent and let automatically execute it after the person has died. So, this will help to decrease the obligation of the inheritors of the dead author person in order to clean up existing contracts such as for example a lease contract associated with the apartment where the author person lived, or any other contracts, memberships, which costs money regularly, abonnement contracts for newspapers, memberships in whatever associations, clubs, societies etc.

As to the notification workflow it should be noted that the workflow beginning with the mortician person or the physician who determines the occurrence of an author person having died is very direct, fast and free of ambiguities, because for example when the author person is identified by nationality and personal passport number, each physician or mortician involved is obliged or can be obliged to communicate a "mortal event message" immediately to the inventional portal server including nationality and passport number (and name if required).

So for example the mortal event is easily made known to the inventional portal system 30 minutes after the author person has died.

According to a specific aspect of this embodiment the death related messages can also be published, for example at a specific portal freely accessible in the internet.

In further modification of this separate aspect, it is also preferred to implement some modification in which only some "peer group" of the author person has access to this website which is protected by a password. The password can than be communicated in a personal message to each member of his peer group. By this, even messages having a larger content such as a blog with a huge plurality of image, audio or video data can be accessible to the desired recipient's group.

Further, it should be noted that the messages of an author person will only be sent after the author person is dead if the author person has once successfully been identified to the system.

### 3. BRIEF DESCRIPTION OF THE DRAWINGS

The present invention is illustrated by way of example and is not limited by the shape of the figures of the drawings in which:
Figure 1 is a schematic depiction illustrating an overview of the main system components involved in the inventional method according to a preferred embodiment thereof, in which the messages are death related messages and the trigger signal for sending the messages is generated by a mortician/physician;
Figure 2 is a schematic depiction of the control flow during the registration phase of this embodiment;
Figure 3 is a schematic depiction illustrating the deposition phase and the deposition of a new order;
Figure 4 is a schematic depiction according to figure 3 illustrating the changing of an existing deposition order;
Figure 5 is a schematic depiction of the control flow of the inventional method of this first embodiment during the notification phase;
Figure 6 is a schematic depiction of the logical structure of the client-related data provided in the database of the portal server;
Figure 7 is a schematic depiction according to figure 1 according to a preferred further embodiment thereof, in which a message is a warning message and the trigger signal for sending the messages is generated by an elapse of a timer implemented in a network-coupled device, here a mobile phone, which also sends the trigger signal to a notification server; and
Figure 8 is a schematic depiction according to figure 1 and according to a preferred further embodiment of the inventional method, in which a pre-defined standard message is nearly automatically sent by the police to the family of a victim.

### 4. DETAILED DESCRIPTION OF THE PREFERRED EMBODIMENT

According to a preferred embodiment of the present invention. The inventional method is mostly implemented in a portal server 110 which is connected via the internet or other network connection with a plurality of clients 105, each communicating with an adequate browser tool. Further, the portal server 110 is connected to a mortician network 115, also via the internet, or telephone connection or the like in order to get trigger signals from basically all over the world, indicating that a certain person has recently died.

Further, the portal server 110 is connected in a local area network (LAN) - see the dotted frame - with a notification server computer 120 which implements the basic steps of sending away the electronic messages which have been predefined by the registered clients 105, individually.

Further, the portal server 110 is connected also via a local area network connection to a so called "physical things delivery server" 125 which is a server computer which implements a workflow to send away physical things being parcels or envelopes or the like as they have been described above, also only in case the death-related trigger signal comes into the portal server 110.

Further, an electronic media delivery server 130 is implemented and connected also in the local area network connection to the portal server and the notification server 120, which performs a respective sending away of death-related messages which have been registered in advance by a client 105.

Then basically the recipients 135A, 135B and 135C are depicted on the right side of the drawing illustrating schematically the plurality of electronic devices which act as receive devices for the death-related electronic messages. Recipients 135A are also to be understood as other people personally which are addressed by there individual seat addresses and which are deemed to receive also the physical things sent out by the physical things delivery server 125.

The portal server 110 is a hardware/software combination offering an internet portal which is meant to be basically public, offering a public access for exploring the services offered by the portal and offering a private access, password protected to individual registered persons.

Each client 105 may reserve some storage space in a database 112 for storing particular electronic messages having a predefined content and predefined recipient addresses.

The recipient addresses should be held up-to-date. A means for doing that may be implemented by a mechanism which regularly prompts the user to check their validity. This prompt may be implemented by e-mail, or by some pop-up prompt message automatically starting when the user logs-in into the portal.

Electronic messages can for example be e-mail-messages addressed to a predefined e-mail-address or short messages (SMS) or multimedia messages (MMS) addressed to a predefined mobile phone number of a recipient or other electronic messages which maybe popular and vastly used in near or further future.

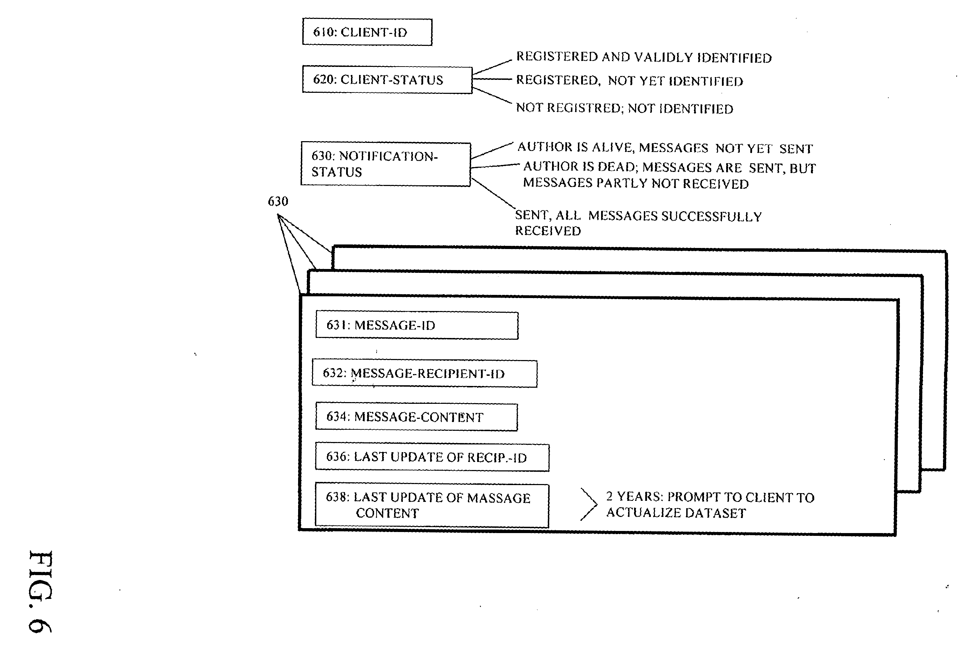

The storage space provided for each client 105 is structured according to figure 6 for example. Each client has a client ID 610, for example a unique country code followed by the passport number and followed by last name and first name of the client. Further, each client has associated a client-status field 620 which reflects the business status of the client. For example the status field can have the following values:

### Status 000:

The client has not been registered and has not been identified.

### Status 001:

The client has been successfully registered with the system but has not yet been successfully identified to the system.

### Status 011:

The client has been successfully registered and validly or successfully being identified to the system.

Further, each client ID has some storage space reserved for recording and storing messages to be sent out as so called "death-related-messages" to the desired message recipients after the trigger signal has been successfully received and evaluated as to be validly true at the portal server. The electronic messages stored in this storage space have the following logical structure:

A message ID field 631 is provided in order to give to each message a unique identifier, regardless of the client ID 610.

A message recipient ID field 632 stores the e-mail-address or the mobile phone number of the respective desired message recipient device, i.e. a computer in the sphere of the recipient person, or the mobile phone of the desired recipient person. Further, a message-content field 634 is reserved for each electronic message. The size of this field depends strongly of the kind of e-mail message. If the e-mail message contains only text, this will be quite small in size. If the message content however contains graphics-data or audio-data or even video-data, than the size is respectively larger.

Further, optionally, there is provided a field 636 for storing the date of the last recent update of the recipient ID stored in field 632 which is evaluated regularly for prompting the user to check its validity.

Further, optionally, there is provided a field 638 for storing the date of the last recent update of the message content stored in field 634.

The notification server 120 comprises a multi-functional communication interface in order to be able to receive messages of any existing type. For example the notification server 120 comprises an e-mail send/receive interface, a telephone interface and a mobile phone interface for receiving short message services (SMS) or multimedia messages (MMS). Further, optionally, there is a person whose job is to take care of the incoming messages and answering also incoming telephone calls in order to receive and evaluate the above mentioned death-related-trigger signals associated with the death-event of a client 105.

Further, the notification server has LAN connections to both, the physical thing delivery server 125 and the electronic media delivery server 130. Further, an evaluation logic is implemented in the notification server implementing some workflow which evaluates any incoming death-related trigger signal and some workflow for performing plausibility tests for verifying if the message sent by the mortician network and saying that a specific person is dead, is true or not.

The electronic media delivery server 130 is a software/hardware combination, i.e. a computer including some program implementing a workflow for processing an incoming command of the notification server ordering the delivery of the messages associated with a particular client ID which has been successfully verified by the notification server workflow as indicated above. The electronic media delivery server extracts the client ID from the order of the notification server, accesses the database 112 by means of the client ID and selects all messages stored with this client ID. After the selection, the messages are sorted by the transmission medium, i.e. all e-mail messages are grouped together, all mobile phone addressed messages (SMS, MMS) are grouped together. Then, in a preferred embodiment of this method those groups of messages are enqueued for being sent out for transmission via the respective networks. In order to do that, the electronic media delivery server is provided with respective input and output interfaces.

Then the selected messages are sent over the respective electronic network, and respective receipt confirmation messages are received in return to a successful receipt at the recipient. Those confirmation messages are evaluated and for each message the receive status is stored associated with the client idea and associated with the respective message ID. So, for example when a message for successfully receipt at the recipient side comes in, the respective receipt status field value is set to a respective predefined value encoding this successful status. In case, the message was sent, but not received successfully, a respective different value is set, representing the unsuccessful transmission of the massage and otherwise, the receipt status remains in a initial state, namely "message not yet sent" which value is set after the message was stored at the portal server successfully.

The physical things delivery server 125 is a server computer implementing a programmed workflow similar to that one of server 130, but in which workflow to an incoming client ID the respective physical things stored to this client ID are selected physically and transported to an output compartment, after the receiver address is confirmed to be obviously okay. Then, the parcels or envelopes, i.e. the physical messages are sent out by postmail at the same day or the next day. Also here, when a parcel or a letter is coming back because a proper recipient could not be determined the address indicated at the letter/parcel, than those parcels/letters are collected in a separate deposition room. Depending of a prescribed workflow, these things are conserved for a predefined plurality of years and then destroyed.

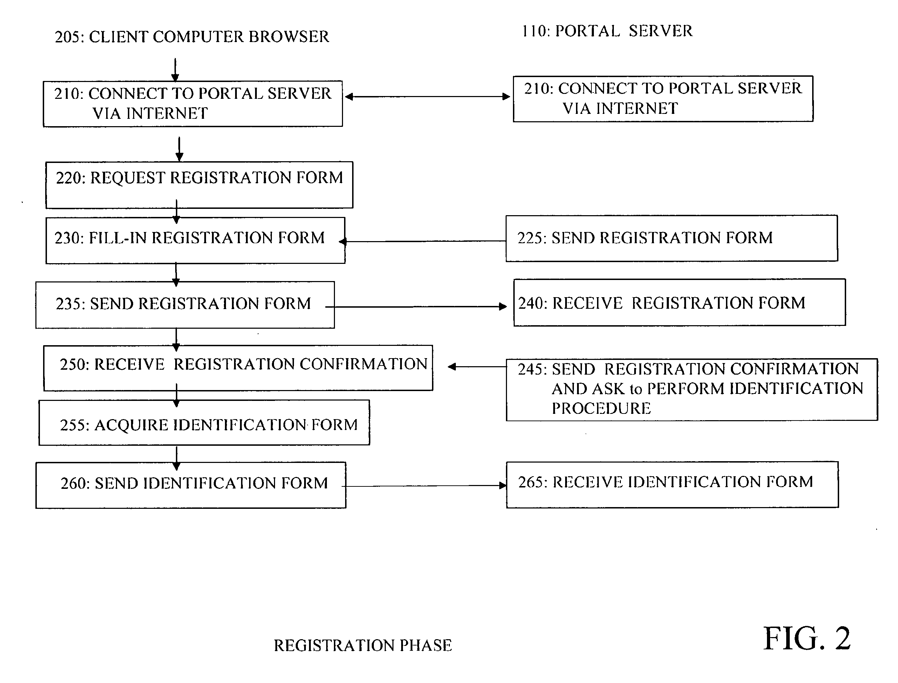

Figure 2 is a schematic depiction of the control flow during the registration phase of this embodiment;

With reference now to figure 2 the workflow between a client computer browser and the portal server during the registration phase for the client will be described in more detail next below.

In first step 210 the client person 105 uses its browser in order to connect to the portal server via the internet, simply by addressing the URL of the portal server. The portal server responses to this connect request by displaying the website HTML code to the user in its browser, step 215.

In a next step 220 the client person requests an online registration form which has been set up in advance in order to provide the legal requirements to be registered as a registered user of the portal services. In response the registration form is sent to the client browser in a step 225.

Then, in a next step 230 the user person fills-in the registration form by indicating all data which is normally required for a user to type in order to begin a workflow in which services can be consumed by the user and the services are paid by the user. So for example prename, last name, address, telephone number, e-mail-address, mobile phone number, bank account data and the like, are collected.

Then in a next step 235 the user sends the complete registration form to the portal server which receives it in a step 240.

After the usual plausibility checks and completeness checks have been successfully performed at the portal server by respective program steps, the portal server sends a registration confirmation message to the client 105 computer browser including an additional prompt or ask to perform an identification procedure, such as for example the "Post Ident" procedure as known in prior art in Germany, where a person fills-in some crucial personal data into a prepared form and lets this filled-in form or authenticate at an official institution, such as a post office. The crucial personal data are in particular the following:

Last name, prename, address as appearing from the client users identification card, further the passport number or the identification card number, valid bank account data, and other data, if ever acquired for a useful business workflow.

After the officer has confirmed the authenticity of the data by his personal signature and by an official office stamp, the user has acquired the identification form, step 255.

In a next step 260 the user sends the original identification form via post mail to the post address associated with the portal server 210, step 260, and at the portal side the identification form will be received in a step 265. Then, after the authentication has been checked by a staff member at the portal server, this staff member selects the data set of the respective user and qualifies the user as a registered and identified user in the respective database 112 by setting respective client status fields to respective values. Details are given in figure 6 as to the field 620 concerning the client status.

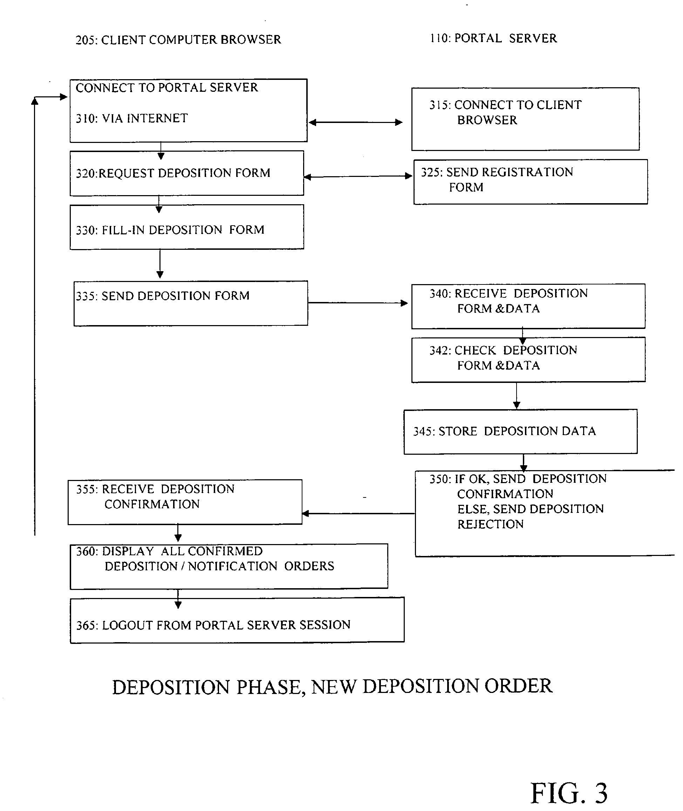

Figure 3 is a schematic depiction illustrating the deposition phase and the deposition of a new order;

With new reference now to figure 3 the workflow of creating a new deposition order for depositing an electronic message comprises a first step 310 of connecting to the portal server via the internet. In this embodiment a user must be a registered user and a validly identified user for completing this workflow. So he connects to the portal server as described before with reference to figure 2, step 315 and requests a deposition form. According to this preferred embodiment the deposition form is only sent to the user when the status field of the registered user has the value corresponding to be validly identified. If the value is different, the request 320 is refused by the server.

Then, in a next step 330 the user fills-in the deposition form. The deposition form comprises input fields for the message content 634 and input field for identifying the address, where the electronic message has to be sent. Thus, this is a message recipient ID 632. In case the electronic message will be an SMS-message, the recipient ID 632 is the mobile phone number of the recipient persons mobile phone device. In case the electronic message is an e-mail message, field 632 should be filled in with the respective e-mail address of the recipient person. So for example the message content field 634 comprises the following text:

"Hello John, I am dead now, sorry for having talked not earlier to you. But I was ashamed of doing that. I want to tell you that you have always been my best friend. Take care of you and we see us in heaven. Yours sincerely Jacky."

Then in step 335, after fields 632 and 634 are filled with useful values, the user pushes a "sent message button" provided to him in order to send the deposition order to the portal server.

At the portal server the respective dataset will be received and interpreted by a plausibility engine, which checks the correct form of a mobile phone number or that of an e-mail address by analyzing the grammar of the content of field 632, and checks if the message content of field 634 if it is at least not empty.

If the check procedure 342 containing further plausibility checks results that all is okay, the message content along with the recipient ID is stored as a deposition dataset in a step 345. In order to retrieve the message later for sending it out when the author of the message is dead, the system creates a message ID and stores this along with the deposition data in the same data set, but in a separate field 631.

Then, in step 350, a message is sent back to the client user. This is a deposition confirmation if the check of step 342 was okay and the deposition data set was successfully stored, and otherwise a rejection, optionally including an automatically generated reasoning for the rejection. The reasoning may for example comprise a notification that the mobile phone number is incomplete, or wrong, i.e. if the number is not associated with any person, depending on the extent to which the plausibility check of the target address has been performed. It should be noted, that the plausibility test may include to dial automatically the indicated telephone number or to sent a testmail in order to know if the target address or target telephone number is basically valid and will work properly.

Then, in step 355 the client user receives this feedback message (confirmation or rejection). Then the user may choose the option in step 360 to display all confirmed deposition orders he has registered successfully with the portal server according to this workflow.

Further and finally in step 365, the user logs-out from his portal server session.

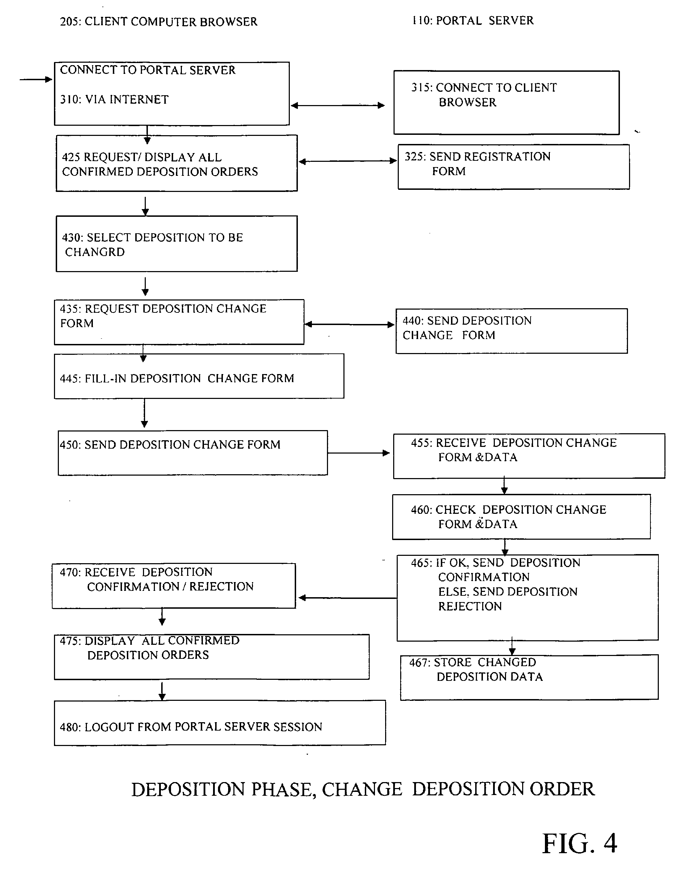

Figure 4 is a schematic depiction according to figure 3 illustrating the changing of an existing deposition order;

With reference now to figure 4 an exemplary workflow illustrating the changing of an existing deposition order begins again with a step of connecting to the portal server from the client browser, see steps 410 and 415. Then, in step 425 the client user pushes the button for displaying all confirmed deposition orders. This request is received at the portal server, and a respective database access will be initiated, wherein for this particular client all valid deposition datasets are sent back to the client browser.

In step 430 the client user may view all of those orders and may select a particular one for being changed.

So the user requests a deposition change form in step 435 which is evaluated and sent back to the user in step 440.

Then, in step 445 the user may edit the text of the content of the respective electronic message until he is satisfied with the new, changed content. Then he may also update the mobile phone number or the e-mail address of the desired message recipient, if he knows that such update is necessary.

Then, in a further step 450 the user sends the deposition change form to the portal server, where the changed dataset is checked for correctness and plausibility as described before with reference to step 340, 342 and 345 with reference to figure 3, see figure 4, steps 455, 460. Then, the changed deposition dataset is stored in a new dataset assuming the changed data have past successfully the plausibility checks. Again, a confirmation or rejection message is sent to the client, step 465 which is received at the client side in a step 470.

Further, in step 475 the user may display all confirmed deposition orders and may then log out from his portal server session.

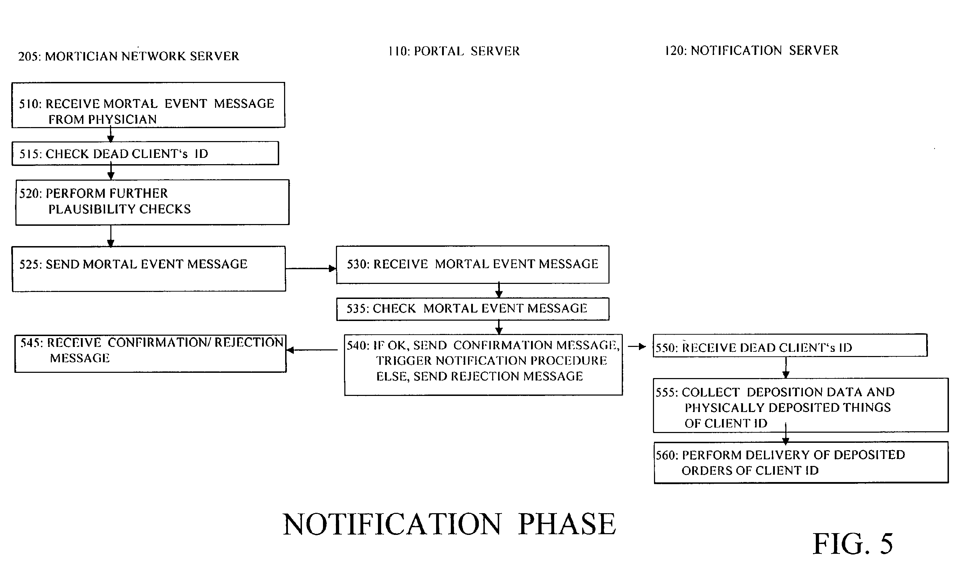

Figure 5 is a schematic depiction of the control flow of the inventional method of this first embodiment during the notification phase.

In a first step 510 of the workflow during the notification phase the mortician is assumed to be called to a hospital in which a person has actually died. The mortician is assumed to speak with the physician where is determined that the person is actually dead. The mortician receives a copy of the death certificate of the dead person - in the inventional context the author of a couple of messages stored at the portal server. The mortician knows thus about the name, the passport number and the address of the dead person or is at least able to explore the passport number which is taken in this preferred embodiment for identifying a person. So, the mortician checks the dead client's ID in a step 515. Then, a couple of further predefined plausibility checks will follow including the test if or if not the dead person is a registered user and validly identified user of the portal server application just described above, step 520. In case the dead person has no relation to this portal, the usual prior art workflow will be followed by the mortician.

In the case, however, the dead person is a registered user at the portal, the mortician sends a "mortal event message" to the portal server 110, step 525, indicating the author would now be dead. Of cause this message comprises the personal ID of the dead person which is also known at the portal server. For example the personal ID may be the last name, one first name and then followed by the country code and the actual passport number or identification card number.

This mortal event message is sent by either of the following transmission types:
Secure e-mail communication, secure SMS communication or secure MMS communication, or by telephone but advantageously only performed with trusted persons. In a phone communication a secure key should be exchanged in order to make sure that there is no fraudulent attempt to provoke a wrong mortal event message.

In a step 530 the mortal event message is received at the portal server and is checked according to a predefined additional workflow including automatic, computer programmed steps and - if necessary - also human intervention.

In particular, the status field of the client, field 620 in figure 6 is checked, after a client ID has been properly confirmed to be true and matching only a single person. The receipt of the mortal event message is confirmed back to the mortician network or is rejected as invalid mortal event message if any of the data sent from the mortician to the portal server is not concise or is obviously incorrect. Also, if there is no client having such client ID registered in the database of the portal server this is optionally notified back to the mortician network. This helps in cases where more than one portal server exist in the world which offer basically the same services. This is done in a step 540.

In step 545 the confirmation or rejection message respectively, is received at the mortician network server.

On the other hand, after a mortal event message has been confirmed to be correct and after it has been successfully checked that a user is in the status to be registered and validly identified (see field 60 of figure 6) the actual notification procedure is triggered by the portal server also in step 540, wherein the client ID is sent to the notification server 120 via a LAN network connection in order to trigger the start of the workflow which is related to the sending of the death-related messages to the predefined message recipients.

So, in a first step 550 the client ID of the freshly dead person is received at the notification server. Both, portal server 110, and notification server 120 have access to the centralized database 112 storing all relevant data including the electronic messages.

So, in a further step 555 the notification server starts an automatic database access procedure which collects all deposited electronic data and selects all physically deposited things of the transmitted client ID. The electronic messages are grouped by electronic transfer medium and are then sent away via the respective electronic network. So, actually, e-mail messages to the predefined e-mail recipient person are sent away and short messages (SMS or MMS) are sent away via respective mobile phone networks.

Further, the selected physical things which had been deposited at the client are sent away via traditional physical post mail services. This is understood as step 560, of performing the delivery of the deposited orders associated with a certain client ID.

Figure 6 is a schematic depiction of the logical structure of the client-related data provided in the database of the portal server.

Next and with reference to figure 6 the logical structure of the client-related data are described in more detail and with reference to a specific embodiment of the inventional method, as follows:

Field 610 is designed for storing the ID of a client person. This may be a string of characters including country code and passport number, further including name information such as the sequence of last name and first name. In a different embodiment this may also be a hash code obtained by a hash function which encodes one ore more of the personal information above and which is additionally enriched by some random component generated by a random generator. Encoding the client ID by some secret hash function involves the additional advantage that this personal, private identification data can not be misapplied or abused for criminal activities.

Next, the client - status field 620 - will be described in more detail for a specific embodiment thereof:

The initial value of this data field is "not registered, not identified". The next status is "registered and not yet identified", which value is taken when the initial value is overwritten after a successful registration procedure for a certain user.

Then, after the user has himself validly identified to the system, the status field is changed to "registered and identified". Concurrently to entering this state a further notification status field 630 is changed from zero state representing an undefined state to the state of "nothing yet deposited". This state indicates that the user has actually no valid deposition orders brought into the system.

After the user has successfully run through the deposition workflow and has deposited some electronic message, the notification status field 630 is changed to "something deposited but not yet sent". The status field will remain in this state as long as the user does not delete his electronic messages which were deposited before and until he will be notified as "dead" by the mortician network server, see back to figure 5. So it can be assumed, that this is basically the most important and most frequently used status.

After the mortal event message has been successfully received at the portal server in case the respective author person has died, the messages are sent away, and the status of field 630 is changed to "author is dead, messages are sent but not received". This is actually the state when the electronic messages and the physical messages have been sent away. After a receipt confirmation signal has been received at the notification server associated with an electronic message the state may be changed to "sent and successfully received by the recipient". Depending on a respective concrete implementation this status field can be associated with a client ID and has then the meaning for representing a general information on all of the existing messages and physical things, or it can also be maintained each message-based, i.e. such status field can be provided with each and every message in order to reflect the status specifically for each message and physical things parcel.

In case, a cash money transfer or a stocks transfer is to be done according to some message, the before-described lawyer is informed to control the sending of the messages and to avoid illegal transactions of money or stocks.

With respect to the logical data structure of an electronic message there is a message ID field 631, a message recipient ID field 632, a message content field 634.

Further, optionally, there is provided a field 636 for storing the date of the last recent update of the recipient ID stored in field 632.
Further, optionally, there is provided a field 638 for storing the date of the last recent update of the message content stored in field 634.
By means of monitoring the date of these fields and comparing the dates to the actual date, the control program at the portal server may prompt the author client user who ordered an electronic / physical message to verify this data and to update this data if necessary. Such prompt can also be sent via e-mail to the author client.

These fields are provided for each electronic message 630. Also for physical messages a respective dataset is provided which is generated in a program dialogue between the user and the portal server, or also by automated e-mail correspondence.

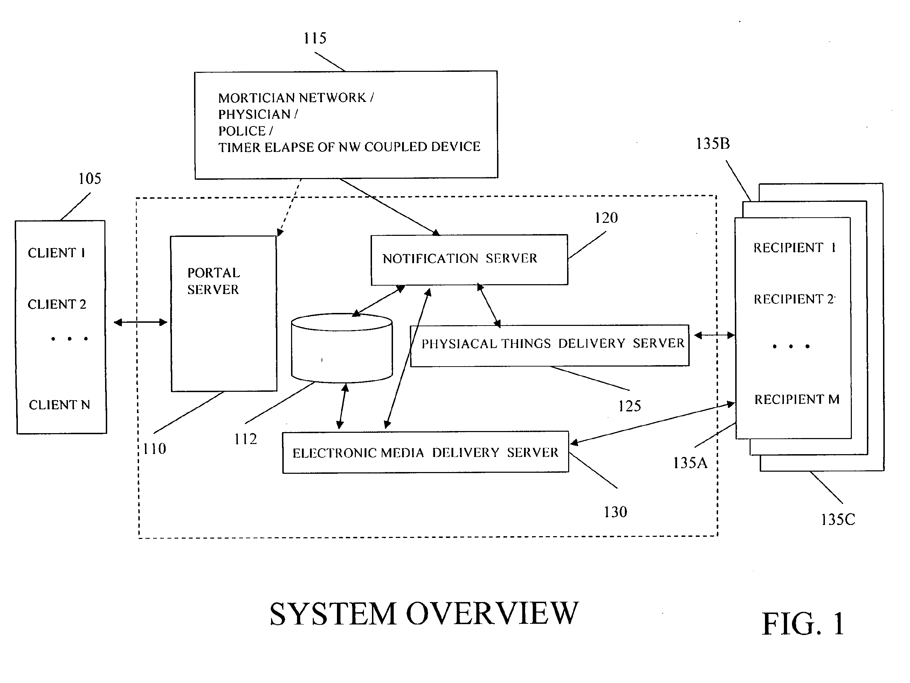

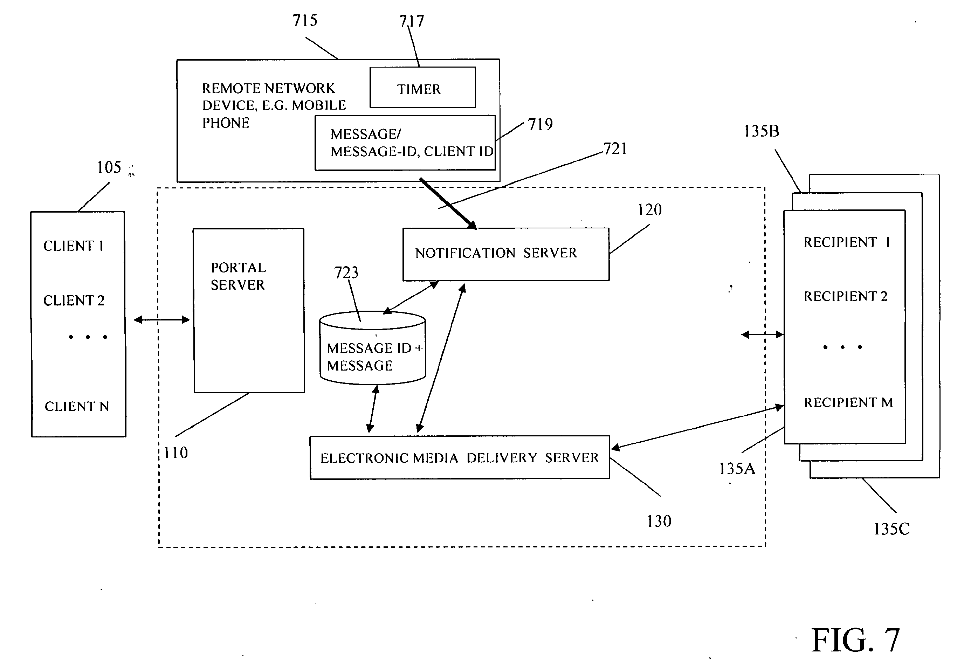

Figure 7 is a schematic depiction according to figure 1 and according to a preferred further embodiment thereof, in which a message is a warning message having some highly important content such as mentioned above in the example of a murderer and a threatened witness.

This message is stored according to the embodiment described before, after the author client 105 of the message has successfully registered with the portal server 110.
The event in the above sense is the elapse of the timer which has been pre-programmed with date and time. Further, the event signal comprises the sending of a dataset 719 which contains at least a message ID which has been registered with the notification server far in advance to the event and according to, or at least logically similar to the first preferred embodiment.

The network coupled device, here a mobile phone 715, sends the event signal comprising the client ID and message ID to the notification server 120 via wireless transmission 721.

After receipt at the notification server, this server looks up a database 723 coupled to it for retrieving the message to be sent by scrolling the message ID which is used as an index into a respective table of the database. After the message has been extracted from the database, it is sent electronically to the desired recipient whose address is also stored with the message.

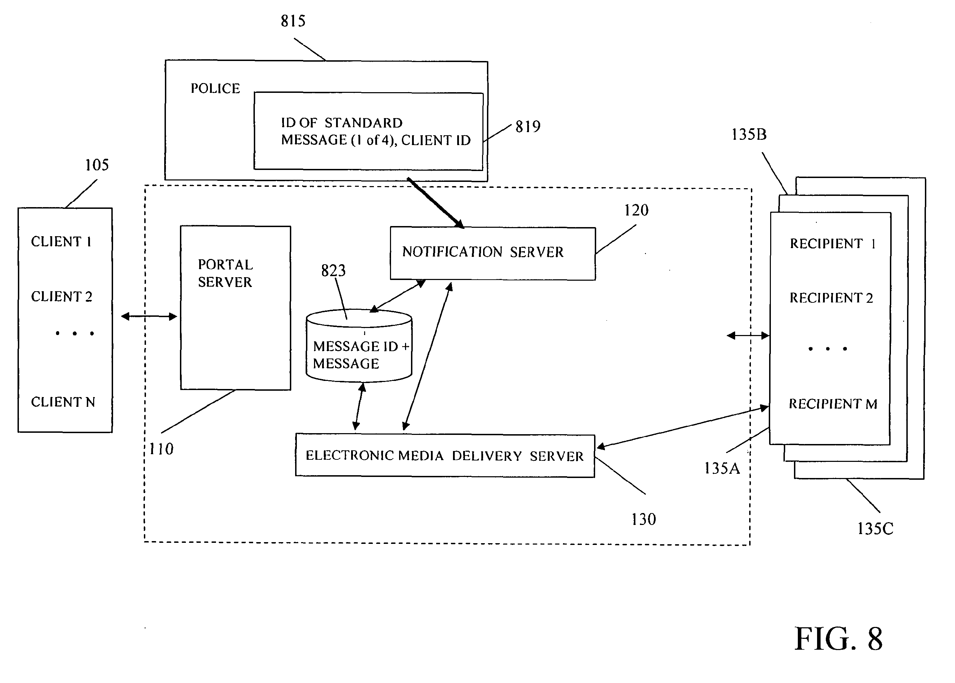

In a further preferred embodiment of the inventional method which is illustrated by figure 8, the police is significantly helped to inform the family of a victim in case of an accident in which many persons are involved as victims.

Figure 8 is a schematic depiction according to figure 1 and according to a preferred further embodiment of the inventional method, in which a pre-defined standard message is nearly automatically sent by the police 815 to the family of the victim.

During an initialization process each person who decides to form part of such process registers at the portal server 110 indicating at least one electronic address of a family member which is desired to be informed according to the inventional method in case the person would be involved in an accident.

For example, four different standard messages are provided as follows:
1) "The person is involved in an accident and is only slightly injured"
2) "The person is involved in an accident and is seriously injured, but not immediately menaced by death"
3) "The person is involved in an accident and is heavily injured and menaced by death"
4) "The person is involved in an accident and has died due to his injuries"

The personal ID of the registered client is for example his passport number.
In case a bigger catastrophic accident happens in which the registered client is involved, the police just needs to enter the victim's passport number into a respective predefined communication form, select the degree of injuries ( 1) or 2) or 3) or 4) ) and send a dataset 819 derived uniquely by the form to the notification server computer 120.
The dataset may look like:

A1..A20 indicating the person's ID, 2 indicating 2^{nd} level injuries.

The server computer 120 will receive that dataset and will retrieve the address of the registered family member stored with the victim's ID in the database 823, and will send out a predefined message to the wife or husband or the relatives of the author (which are registered before) informing the wife that something had gone wrong with the person. This message will contain clear text and may contain the clear text the name the victim and the clear text of the victim's degree of injuries. Further, the message can be enriched by the address of the hospital where the victim is brought to.

Then it is easy for the family to just answer this message in order to request for further details or to address the hospital readily. Further, the police may in turn send an accident report enriched by further details, photographs, etc about the person and the accident. Advantageously the workflow is nearly fully automatically running and so the police is able to process many "accident notifications" in a relatively short time. By that the handling of notifications is significantly improved.

The invention can take the form of an entirely hardware embodiment, an entirely software embodiment or an embodiment containing both hardware and software elements. In a preferred embodiment, the invention is implemented in software, which includes but is not limited to firmware, resident software, microcode, etc.

Furthermore, the invention can take the form of a computer program product accessible from a computer-usable or computer-readable medium providing program code for use by or in connection with a computer or any instruction execution system. For the purposes of this description, a computer-usable or computer readable medium can be any apparatus that can contain, store, communicate, propagate, or transport the program for use by or in connection with the instruction execution system, apparatus, or device.

The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W) and DVD.

A data processing system suitable for storing and/or executing program code will include at least one processor coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution.

Input/output or I/O devices (including but not limited to keyboards, displays, pointing devices, etc.) can be coupled to the system either directly or through intervening I/O controllers.

Network adapters may also be coupled to the system to enable the data processing system to become coupled to other data processing systems or remote printers or storage devices through intervening private or public networks. Modems, cable modem and Ethernet cards are just a few of the currently available types of network adapters.

## Claims

1. A communication method implemented in an electronic network portal server computer (110) for sending a message of a respective message author person (105) to at least one message recipient (135) in response to an incoming event signal associated with said author person, comprising the steps of:
a) prior to the income of said event signal, storing (345) said message of said author person, along with a respective address of at least one addressee acting as a future recipient (135) of said message,
b) waiting for receiving said event signal, wherein said event signal triggers the start of sending said message,
c) receiving (510) said event signal accompanied with an ID (610) of said author person,
d) evaluating (515, 520) said event signal and said ID (610) for retrieving said stored message, and
e) sending (560) said message to said recipient,
f) said method being **characterized by** said event signal being selected to be one of the group of:
f1) a signal generated by elapse of a pre-programmed timer of a network device located remote from a respective sender server computer and associated solely with a single author, wherein said network device is by itself able to establish a communication channel to said server (110, 120) and to transmit said event signal to said server along with a pre-programmed ID (610) of said author person (105),
f2) a signal generated by an authority, in particular by the police in case of an accident in which said author person (105) is involved,
f3) a signal generated by a mortician network or by a physician telling that said author person (105) has been found dead,
f4) a signal generated by a bank telling that a predefined amount of money has been drawn from or credited to a bank account of said author person (105).

2. The method according to claim 1, feature f3), further comprising the steps of:
a) storing a parcel of said author person, along with a respective address of an addressee acting as a future recipient person of said parcel,
b) waiting for receiving said predefined event signal associated with said author person, wherein said event signal triggers the start of sending said parcel,
c) receiving said event signal accompanied with an ID of said author person,
d) evaluating said event signal_and said ID for retrieving said stored parcel, and
e) sending said parcel to said recipient.

3. The method according to claim 1, feature f3), further comprising the step of sending a message after a predetermined elapse of time after said event signal has come in.

4. The method according to claim 1, feature f3), further comprising the step of publishing a message at a predefined website.

5. The method according to the preceding claim, wherein said website is protected by a password which is sent in a message according to a method according to claim 1 feature f3).

6. An electronic data processing system implemented in an electronic network portal server computer implementing a communication method for sending a message of a respective message author person to at least one message recipient in response to an incoming event signal associated with said author person, comprising:
a) means (112) for storing said message of said author person, along with a respective address of at least one addressee acting as a future recipient (135) of said message,
b) means for receiving said event signal accompanied with an ID of said author person,
c) means (120) for evaluating said event signal and said ID and for retrieving said stored message, and
d) means for sending said message to said recipient,
**characterized by**
having a notification control status field (630) storing a flag indicating that an author person is alive and messages are not sent, and storing a flag indicating that an author person is dead and messages are to be sent or have been sent.

7. A computer program product implementing a communication method in an electronic network portal server computer for sending a message of a respective message author person to at least one message recipient in response to an incoming event signal associated with said author person, comprising a computer useable medium including a computer readable program, wherein the computer readable program includes a functional component that when executed on a computer causes the computer to perform the steps of:
a) prior to the income of said event signal, storing said message of said author person, along with a respective address of at least one addressee acting as a future recipient of said message,
b) waiting for receiving said event signal, wherein said event signal triggers the start of sending said message,
c) receiving said event signal accompanied with an ID of said author person,
d) evaluating said event signal and said ID for retrieving said stored message, and
e) sending said message to said recipient,
f) said method being **characterized by** said event signal being selected to be one of the group of:
f1) a signal generated by elapse of a pre-programmed timer of a network device located remote from a respective sender server computer and associated solely with a single author, wherein said network device or a user thereof is able to establish a communication channel to said server and to transmit said event signal to said server along with a pre-programmed ID of said author person,
f2) a signal generated by an authority, in particular by the police in case of an accident in which said author person is involved,
f3) a signal generated by a mortician network or by a physician telling that said author person has been found dead,
f4) a signal generated by a bank telling that a predefined amount of money has been drawn from or credited to a bank account of said author person.
